# EUROPEAN PATENT SPECIFICATION

(11) **EP 1 269 973 B1**
(45) Date of publication and mention of the grant of the patent: **05.10.2005**
(21) Application number: 01917832.6
(22) Date of filing: 05.04.2001
(51) Int. Cl.: A61K 7/06, A61K 31/506, A61P 17/14

(54) **HAIR GROWTH STIMULANT COMPOSITIONS**
HAARWACHSTUMSFÖRDERNDE ZUSAMMENSETZUNGEN
COMPOSITIONS STIMULANT LA POUSSE DES CHEVEUX

(30) Priority: 07.04.2000 JP 2000106500
(43) Date of publication of application: 02.01.2003
(73) Proprietor: TAISHO PHARMACEUTICAL CO., LTD, Tokyo 170-8633 (JP)
(72) Inventor: IMAMURA, Koji, c/o Taisho Pharmaceutical Co. Ltd., Toshima-ku, Tokyo 170-8633 (JP); OCHIAI, Rumi, c/o Taisho Pharmaceutical Co. Ltd., Toshima-ku, Tokyo 170-8633 (JP); OKAJIMA, Takako, c/o Taisho Pharmaceutical Co. Ltd, Toshima-ku, Tokyo 170-8633 (JP); MORIOKA, Susumu, c/o Taisho Pharmaceutical Co. Ltd, Toshima-ku, Tokyo 170-8633 (JP); HORIE, Taro, c/o Taisho Pharmaceutical Co. Ltd., Toshima-ku, Tokyo 170-8633 (JP)
(74) Representative: HOFFMANN EITLE
(86) International application number: PCT/JP2001/002942
(87) International publication number: WO 2001/076541

(56) References cited:
- EP-A1- 0 970 683
- WO-A-99/53923
- JP-A- 6 211 681
- US-A- 5 192 534

## Description

### TECHNICAL FIELD

The present invention relates to a hair growth stimulant composition comprising minoxidil as an active component, and more particularly to a hair growth stimulant composition free from problems such as crystal precipitation and the like at low temperatures.

### BACKGROUND ART

The chemical name for minoxidil is 6-(1-piperidinyl)-2,4-pyrimidinediamine-3-oxide. The adaptation of minoxidil as a hair growth agent is disclosed in USP No. 4,139,619. Due to the excellent hair growth obtained by the topical application of minoxidil, hair growth agents containing minoxidil (hereinafter referred to as "minoxidil preparations") have been widely accepted and their sales volume is record breaking.

To increase the effect of these minoxidil preparations, preparations having a higher concentration of minoxidil have been sought after. Until now, minoxidil preparations having a minoxidil concentration of about 3 to 5% by mass have been easily prepared (Japanese Patent Application Laid-open No. 150211/1988).

Patent document WO99/53923 discloses a hair growth stimulant compositions comprising 10 to 50% by mass of water, minoxidil, further comprising 8 to 30% by mass of a polyhydric alcohol, and having a pH of 5.5 to 6.5 (ex. 5, 7 and 10). These compositions contain 10 or 12% minoxidil.

### DISCLOSURE OF THE INVENTION

However, when a high concentration minoxidil preparation (hereinafter referred to as "high conc. minoxidil preparation) contains 10 to 50% by mass of water in order to improve feeling during use, crystal precipitation occurs in the preparation if stored for a long period of time at a low temperature. Therefore, because redissolving the precipitated crystals takes time, problems occur when the hair growth composition is used in cold regions or when stored in cold places.

For this reason, a stable high conc. minoxidil preparation wherein crystal precipitation does not occur even if the preparation is stored at a low temperature has been required. Accordingly, an object of the present invention is to provide such a preparation.

As a result of diligent research on a solvent composition and the like of a high cone. minoxidil preparation to solve the above problems, the present inventors have discovered that by selecting a specific polyhydric alcohol and using it at a specific concentration as a solvent and adjusting the pH of the solution within a fixed range, a minoxidil solution in which a high concentration minoxidil is combined with a fixed amount of water does not precipitate crystals and minoxidil itself is stable, thereby completing the present invention.

Namely, an object of the present invention is to provide a hair growth stimulant composition comprising 10 to 50% by mass of water and 3 to 6% by mass or more of minoxidil, further comprising 8 to 30% by mass of one or more polyhydric alcohols, and having a pH of 5.5 to 6.5.

### DETAILED DESCRIPTION OF THE PREFERRED EMBODIMENT

The hair growth stimulant composition of the present invention contains 3 to 6% by mass or more (hereinafter simply referred to as "%") of the active component minoxidil.

As the solvent for dissolving the minoxidil, a mixture of polyhydric alcohol and water is used. It is required that the final composition comprises 10 to 50% of water and 8 to 30% of polyhydric alcohol. If either one of the polyhydric alcohol or water is not present, preventing the precipitation of crystals is difficult. Also, even if both are present, it is difficult to produce a composition that provides a good feeling during use and effective in suppressing the precipitation of crystals at low temperatures if they are present in an amount outside the above ranges. The present invention has a greater effect when the amount of water is 18% or more because the precipitation of crystals occurs easily. Polyhydric alcohol is incorporated in an amount according to the above range, with 10 to 20% being preferable, and 15 to 20% being particularly preferable.

It is desirable to add ethanol to the above solvent. The final composition preferably contains ethanol in an amount of 70% or less.

As the polyhydric alcohol used in the above solvent, 1,3-butylene glycol, glycerol, dipropylene glycol, Macrogol 400, Macrogol 600, and the like can be given, with 1,3-butylene glycol being preferable. Also, these polyhydric alcohols are preferably used in combinations of two or more types. As particularly preferable combinations of polyhydric alcohols, 1,3-butylene glycol and glycerol combined at a weight ratio of 10:1 to 1:4 and dipropylene glycol and glycerol combined at a weight ratio of 10:1 to 1:4 can be given.

The hair growth stimulant composition of the present invention is prepared by adding 3% or more of the active component minoxidil and other necessary active components and supplementary components to the solvent containing polyhydric alcohol and water, then mixing these components with stirring using a common method. In this instance, it is necessary to adjust the pH in a range of 5.5 to 6.5. If minoxidil is simply added to an aqueous type solvent, the resulting solution is neutral or weakly alkaline. When preparing the high conc. minoxidil preparation using this solution, precipitation of crystals occurs easily at a low temperature. On the other hand, if the solution is on the acid side with a pH of 5 or less, minoxidil itself becomes easily decomposable, even though precipitation of crystals does not occur.

The pH of the hair growth stimulant composition of the present invention is adjusted using a common method. As the pH adjustor, citric acid, hydrochloric acid, lactic acid, phosphoric acid, and the like are preferably used.

Furthermore, as medicinal components that are preferably added to and combined with the hair growth stimulant composition of the present invention, components selected from the group consisting of menthol, vitamin E acetate, pantothenylethylether, hinokitiol, glycyrrhetic acid, and diphenhydramine hydrochloride can be given (hereinafter referred to as "optional components"). Of these optional components, menthol possesses particularly high effectiveness. The addition of menthol as an essential component is even more preferable.

There are no particular limitations to the amount of these optional components to be added. This can be decided experimentally while taking the feeling during use and stability of minoxidil or solvent-type components into consideration. For example, the final composition preferably contains about 0.2 to 0.4% of menthol, a particularly preferable optional component.

In addition to the above components, the hair growth stimulant composition of the present invention may contain various active components and supplementary components normally used in topical preparations in an amount that does not harm the effect of the present invention. Examples of these components include fillers, vasodilators (carpronium chloride, benzyl nicotinate, swertia herb extract, panax ginseng extract, capsicum tincture, and the like), antihistamines (isothipendyl hydrochloride and the like), anti-inflammatory agents (guaiazulene and the like), keratolytics (urea, salicylic acid, and the like), antimicrobial agents (chlorhexidine gluconate, isopropylmethylphenol, quaternary ammonium salts, piroctone olamine, and the like), humectants (sodium hyaluronate, chondroitin sulfuric acid, and the like), extracts of animals and plants (Taxus cuspidata, Paeonia suffruticosa, Glycyrrhisa uralensis, Hypericum erectum, aconite root, Eriobotrya japonica, Artemisia capillaris, Symphytum officinale, Angelica keiskei, Crocus sativus, Gardeniae fructus, Rosmarinus officinalis, Salvia officinalis, Saussurea lappa, Aristolochia debilis, Lupuli strobilus, placenta, and the like), vitamins (retinol acetate, pyridoxine hydrochloride, ascorbic acid, thiamin nitrate, cyanocobalamin, biotin, and the like), anti-oxidants (dibutylhydroxytoluene, sodium pyrosulfite, tocopherol, sodium edetate, ascorbic acid, isopropyl gallate, and the like), solubilizers (diisopropyl adipate, isopropyl myristate, vegetable oils, animal oils, alkyl glyceryl ethers, hydrocarbons, and the like), metabolic activators (panthenol and the like), gelling agents (water-soluble high molecular compounds and the like), adhesives, perfumes, refrigerants (mentha oil, camphor, and the like), dyes, and the like.

However, since the addition of surfactants affects the cutaneous absorption of minoxidil and decreases the feeling during use, it is preferable that the hair growth stimulant composition of the present invention does not substantially contain surfactants.

The hair growth stimulant composition of the present invention thus obtained can be used as a suitable topical preparation such as a lotion, aerosol, tonic, cream, ointment, gel, and the like.

### EXAMPLES

The present invention will be described in more detail by examples, which should not be construed as limiting the present invention.

### Example 1

3.0 g of minoxidil, 10.0 g of 1,3-butylene glycol, 35 g of ethanol, and 20 g of purified water were mixed and stirred until dissolved. A suitable amount of citric acid was added to adjust the pH of the solution to 5.5. Purified water was added again to obtain 100 ml of a lotion preparation (product of the present invention 1).

### Example 2

Using the components shown in Tables 1 and 2, lotion preparations (products of the present invention) were prepared in the same manner as in Example 1. Using the components shown in Table 3, lotion preparations (comparative products) were prepared in the same manner as in Example 1.

### (Composition)

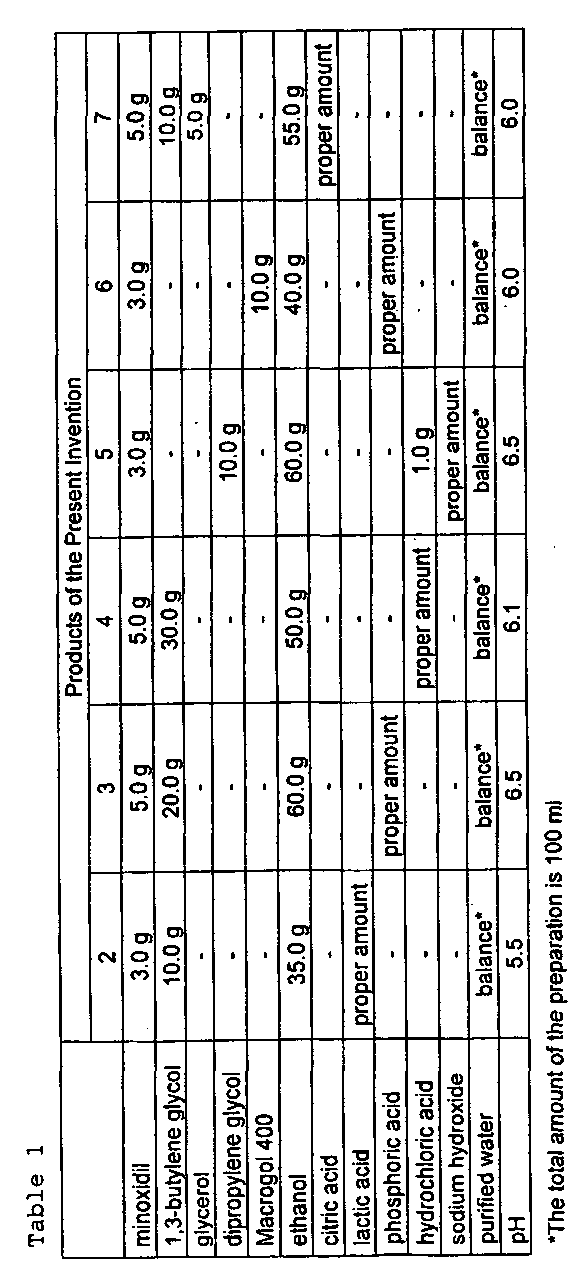

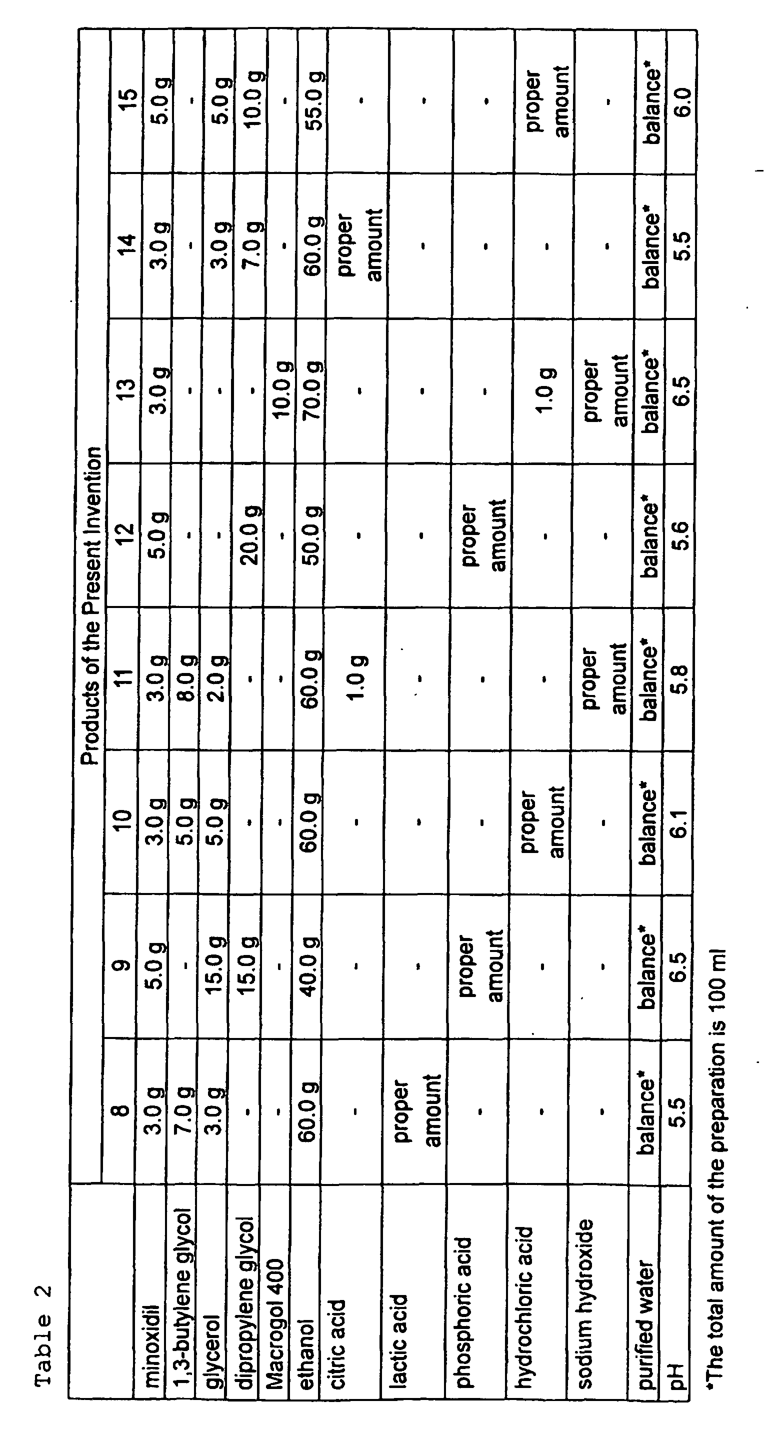

**Table 3**

| | Comparative Products | |
|---|---|---|
| | 1 | 2 |
| minoxidil | 3.0 g | 5.0 g |
| 1,3-butylene glycol | 5.0 g | 10.0 g |
| glycerol | - | 5.0 g |
| dipropylene glycol | - | - |
| Macrogol 400 | - | - |
| ethanol | 45.0 g | 55.0 g |
| citric acid | - | - |
| lactic acid | - | - |
| phosphoric acid | - | - |
| hydrochloric acid | - | - |
| sodium hydroxide | - | - |
| purified water | balance* | balance* |
| pH | 8.8 | 9.3 |

| | | |
|---|---|---|
| *The total amount of the preparation is 100 ml | | |

The products of the present invention and comparative products were stored at -10°C for one week and the degree of crystal precipitation was examined. As a result, the product of the present invention was confirmed to display no sign of crystal precipitation even though the concentration of minoxidil was high. The comparative products were confirmed to display differing degrees of crystal precipitation.

### Example 3

An aerosol was prepared by filling an aerosol can with 30 ml of the lotion prepared in Example 1 and 70 ml of dimethyl ether. In the same manner, an aerosol was prepared using the lotion of Example 2.

### Test Example 1

Lotion preparations of the product of the present invention 16 and comparative product 3 shown in Table 4 were prepared in the same manner as in Example 1. These products were stored under the following conditions and were observed for crystal precipitation for a period of one week. The results are shown in Table 5.

### Storage Conditions:

Storage temperature: -10°C
Container: 35 ml plastic bottle (polyethylene terephthalate)
Amount: 30 ml
Number of bottles used for each observation: 8

**Table 4**

| | Product of the Present Invention 16 | Comparative Product 3 |
|---|---|---|
| minoxidil | 5.0 g | 5.0 g |
| 1,3-butylene glycol | 10.0 g | 5.0 g |
| ethanol | 64.34 g | 64.34 g |
| phosphoric acid | 0.5 ml | 0.5 ml |
| dibutyl hydroxy toluene | 0.05 g | 0.05 g |
| purified water | balance* | balance* |
| water content of the preparation (W/W)% | 18 | 18 |
| pH | 6.0 | 6.0 |

| | | |
|---|---|---|
| *The total amount of the preparation is 100 ml | | |

**Table 5**

| | Number of bottles exhibiting precipitation | | | |
|---|---|---|---|---|
| | Initially | After 1 day | After 4 days | After 7 days |
| Product of the Present Invention 16 | 0 | 0 | 0 | 0 |
| Comparative Product 3 | 0 | 0 | 3 | 3 |

### Test Example 2

Lotion preparations of the products of the present invention 17-20 shown in Table 6 and comparative products 4-8 shown in Table 7 were prepared in the same manner as in Example 1. These products were stored under the following conditions and were observed for crystal precipitation for a period of one week. The results are shown in Table 8.

### Storage Conditions:

Storage temperature: -10 to -16°C
Container: 35 ml plastic bottle (polyethylene terephthalate)
Amount: 30 ml
Number of bottles used for each observation: 8

**Table 6**

| | Products of the Present Invention | | | |
|---|---|---|---|---|
| | 17 | 18 | 19 | 20 |
| minoxidil | 5.0 g | 5.0 g | 5.0 g | 5.0 g |
| 1,3-butylene glycol | 10.0 g | 20.0 g | 30.0 g | 40.0 g |
| ethanol | 49.0 g | 41.0 g | 33.0 g | 24.0 g |
| phosphoric acid | 0.5 ml | 0.5 ml | 0.5 ml | 0.5 ml |
| purified water | balance* | balance* | balance* | balance* |
| water content of the preparation (W/W)% | 32 | 32 | 31 | 32 |
| pH | 5.7 | 5.7 | 5.7 | 5.7 |

| | | | | |
|---|---|---|---|---|
| *The total amount of the preparation is 100 ml | | | | |

**Table 7**

| | Comparative Products | | | | |
|---|---|---|---|---|---|
| | 4 | 5 | 6 | 7 | 8 |
| minoxidil | 5.0 g | 5.0 g | 5.0 g | 5.0 g | 5.0 g |
| 1,3-butylene glycol | 5.0 g | 10.0 g | - | - | - |
| glycerol | - | 5.0 g | - | - | - |
| propylene glycol | - | - | 5.0 g | 5.0 g | 5.0 g |
| ethanol | 53.0 g | 55.0 g | 40.0 g | 60.0 g | 60.0 g |
| phosphoric acid | 0.5 ml | - | 0.5 ml | 0.05 ml | 0.2 ml |
| purified water | balance* | balance* | balance* | balance* | balance* |
| water content of the preparation (W/W)% | 33 | 24 | 53 | 28 | 28 |
| pH | 5.7 | 9.3 | 5.5 | 7.0 | 6.4 |

| | | | | | |
|---|---|---|---|---|---|
| *The total amount of the preparation is 100 ml | | | | | |

**Table 8**

| | Number of bottles exhibiting precipitation | | | |
|---|---|---|---|---|
| | Initially | After 1 day | After 4 days | After 7 days |
| Product of the Present Invention 17 | 0 | 0 | 0 | 0 |
| Product of the Present Invention 18 | 0 | 0 | 0 | 0 |
| Product of the Present Invention 19 | 0 | 0 | 0 | 0 |
| Product of the Present Invention 20 | 0 | 0 | 0 | 0 |
| Comparative Product 4 | 0 | 0 | 1 | 1 |
| Comparative Product 5 | 0 | 1 | 1 | 1 |
| Comparative Product 6 | 0 | 1 | 1 | 1 |
| Comparative Product 7 | 0 | 1 | 1 | 1 |
| Comparative Product 8 | 0 | 0 | 1 | 1 |

## Claims

1. A hair growth stimulant composition comprising 10 to 50% by mass of water and 3 to 6% by mass of minoxidil, further comprising 8 to 30% by mass of a polyhydric alcohol, and having a pH of 5.5 to 6.5.

2. The hair growth stimulant composition according to Claim 1, wherein the polyhydric alcohol is 1,3-butylene glycol.

3. The hair growth stimulant composition according to Claim 1, wherein the polyhydric alcohol is a mixture of 1,3-butylene glycol and glycerol at a weight ratio of 10:1 to 1:4.

4. The hair growth stimulant composition according to Claim 1, wherein the polyhydric alcohol is a mixture of dipropylene glycol and glycerol at a weight ratio of 10:1 to 1:4.

5. The hair growth stimulant composition according to any one of claims 1-4 in the form of a lotion further containing ethanol.

6. The hair growth stimulant composition according to any one of claims 1-4 in the form of a gel further containing ethanol.

7. The hair growth stimulant composition according to any one of Claims 1-6, wherein the incorporated amount of ethanol is 70% by mass or less.

8. The hair growth stimulant composition according to any one of Claims 1-7, wherein the incorporated amount of water is 18 to 50% by mass

9. The hair growth stimulant composition according to any one of Claims 1-8 substantially not containing surfactants.

10. The hair growth stimulant composition according to any one of Claims 1-9 containing citric acid, hydrochloric acid, lactic acid, or phosphoric acid as a pH adjustor.

11. The hair growth stimulant composition according to any one of Claims 1-10 further comprising one or more components selected from the group consisting of menthol, vitamin E acetate, pantothenylethylether, hinokitiol, glycyrrhetic acid, and diphenhydramine hydrochloride.

12. A method for preventing precipitation which comprises incorporating 8 to 30% by mass of a polyhydric alcohol in the composition containing 10 to 50% by mass of water and 3 to 6% by mass of minoxidil, and adjusting the composition to a pH of 5.5 to 6.5.

## Patentansprüche

1. Haarwachstum stimulierende Zusammensetzung, umfassend 10 bis 50 Massen-% Wasser und 3 bis 6 Massen-% Minoxidil, weiter umfassend 8 bis 30 Massen-% eines mehrwertigen Alkohols und mit einem pH von 5,5 bis 6,5.

2. Haarwachstum stimulierende Zusammensetzung nach Anspruch 1, worin der mehrwertige Alkohol 1,3-Butylenglycol ist.

3. Haarwachstum stimulierende Zusammensetzung nach Anspruch 1, worin der mehrwertige Alkohol eine Mischung aus 1,3-Butylenglycol und Glycerin bei einem Gewichtsverhältnis von 10:1 bis 1:4 ist.

4. Haarwachstum stimulierende Zusammensetzung nach Anspruch 1, worin der mehrwertige Alkohol eine Mischung aus Dipropylenglycol und Glycerin bei einem Gewichtsverhältnis von 10:1 bis 1:4 ist.

5. Haarwachstum stimulierende Zusammensetzung nach einem der Ansprüche 1 bis 4 in der Form einer Lotion, weiterhin umfassend Ethanol.

6. Haarwachstum stimulierende Zusammensetzung nach einem der Ansprüche 1 bis 4 in der Form eines Gels, weiterhin umfassend Ethanol.

7. Haarwachstum stimulierende Zusammensetzung nach einem der Ansprüche 1 bis 6, worin die eingefügte Menge an Ethanol 70 Massen-% oder weniger ist.

8. Haarwachstum stimulierende Zusammensetzung nach einem der Ansprüche 1 bis 7, worin die eingefügte Menge an Wasser 18 bis 50 Massen-% ist.

9. Haarwachstum stimulierende Zusammensetzung nach einem der Ansprüche 1 bis 8, im wesentlichen nicht umfassend Tenside.

10. Haarwachstum stimulierende Zusammensetzung nach einem der Ansprüche 1 bis 9, umfassend Zitronensäure, Salzsäure, Milchsäure oder Phosphorsäure als pH-Einstellmittel.

11. Haarwachstum stimulierende Zusammensetzung nach einem der Ansprüche 1 bis 10, weiterhin umfassend eine oder mehrere Komponenten, ausgewählt aus der Gruppe bestehend aus Menthol, Vitamin E-Acetat, Pantothenylethylether, Hinokitiol, Glycyrrhetinsäure und Diphenhydraminhydrochlorid.

12. Verfahren zum Verhindern des Ausfällens, umfassend das Einfügen von 8 bis 30 Massen-% eines mehrwertigen Alkohols in die Zusammensetzung, umfassend 10 bis 50 Massen-% Wasser und 3 bis 6 Massen-% Minoxidil, und Einstellen der Zusammensetzung auf einen pH von 5,5 bis 6,5.

## Revendications

1. Composition stimulant la croissance des cheveux comprenant 10 à 50 % en masse.d'eau et 3 à 6 % en masse dé minoxidil, comprenant en outre 8 à 30 % en masse d'un polyol, et ayant un pH de 5,5 à 6,5.

2. Composition stimulant la croissance des cheveux selon la revendication 1, dans laquelle le polyol est le 1,3-butylène glycol.

3. Composition stimulant la croissance des cheveux selon la revendication 1, dans laquelle le polyol est un mélange de 1,3-butylène glycol et de glycérol à un rapport en poids de 10 : 1 à 1 : 4.

4. Composition stimulant la croissance des cheveux selon la revendication 1, dans laquelle le polyol est un mélange de dipropylène glycol et de glycérol à un rapport en poids de 10 : 1 à 1 : 4.

5. Composition stimulant la croissance des cheveux selon l'une quelconque des revendications 1 à 4 sous la forme d'une lotion contenant en outre de l'éthanol.

6. Composition stimulant la croissance des cheveux selon l'une quelconque des revendications 1 à 4 sous la forme d'un gel contenant en outre de l'éthanol.

7. Composition stimulant la croissance des cheveux selon l'une quelconque des revendications 1 à 6, dans laquelle la quantité d'éthanol incorporée est de 70 % en masse ou moins.

8. Composition stimulant la croissance des cheveux selon l'une quelconque des revendications 1 à 7, dans laquelle la quantité d'eau incorporée est de 18 à 50 % en masse.

9. Composition stimulant la croissance des cheveux selon l'une quelconque des revendications 1 à 8 ne contenant pratiquement pas de tensioactifs.

10. Composition stimulant la croissance des cheveux selon l'une quelconque des revendications 1 à 9 contenant de l'acide citrique, de l'acide chlorhydrique, de l'acide lactique, ou de l'acide phosphorique en tant qu'ajusteur de pH.

11. Composition stimulant la croissance des cheveux selon l'une quelconque des revendications 1 à 10 comprenant en outre un ou plusieurs constituants choisis dans le groupe constitué par le menthol, l'acétate de vitamine E, le pantothényléthyléther, le hinokitiol, l'acide glycyrrhétique, et le chlorhydrate de diphénhydramine.

12. Procédé pour prévenir une précipitation qui comprend l'incorporation de 8 à 30 % en masse d'un polyol dans la composition contenant 10 à 50 % en masse d'eau et 3 à 6 % en masse de minoxidil, et l'ajustement de la composition à un pH de 5,5 à 6,5.
